Europäisches Patentamt

European Patent Office

Office européen des brevets

(11) Publication number: **0 153 171**
A2

## EUROPEAN PATENT APPLICATION

(21) Application number: 85301030.4

(22) Date of filing: 15.02.85

(51) Int. Cl.⁴: **C 07 D 491/04**, C 07 C 91/26, A 61 K 31/47 // (C07D491/04, 317:00, 221:00)

(30) Priority: 16.02.84 HU 60784

(43) Date of publication of application: 28.08.85 Bulletin 85/35

(84) Designated Contracting States: AT BE CH DE FR GB IT LI NL SE

(71) Applicant: CHINOIN Gyogyszer és Vegyészeti Termékek Gyára RT., To utca 1-5, H-1045 Budapest IV (HU)

(72) Inventor: Szentmiklosi, Peter, 75 Rudas L U, H-1064 Budapest (HU)
Inventor: Szuts, Tamas, 53 Csalogany U, H-1027 Budapest (HU)
Inventor: Lengyel, Jozsef, 46 Tel U, H-1043 Budapest (HU)
Inventor: Horvath, Gabor, 5/C Muk L.U, H-1133 Budapest (HU)
Inventor: Marmarosi, Katalin, 19 YBL Miklos Setany, H-2051 Biatorbagy (HU)

(74) Representative: Skailes, Humphrey John et al, Frank B. Dehn & Co. Imperial House 15-19 Kingsway, London WC2B 6UZ (GB)

(54) New 2-hydroxyethyl-trimethylammonium salt of 1-ethyl-3-carboxy-4-oxo-6,7-methylenedioxy-1,4-dihydroquinoline, process for the preparation thereof and pharmaceutical composition containing it.

(57) The invention relates to the new 2-hydroxyethyl-trimethyl-ammonium salt of 1-ethyl-3-carboxy-4-oxo-6,7-methylenedioxy-1,4-dihydroquinoline having ureterodisinfecting activity, which is prepared by reacting 1-ethyl-3-carboxy-4-oxo-6,7-methylenedioxy-1,4-dihydroquinoline with the aqueous or with the aqueous-ethanolic solution of 2-hydroxyethyl-trimethylammonium hydroxyde. The new salt is water-soluble and well resorbed after oral administration. Thus, it is useful both in the human and the veterinary medicine.

This invention relates to the new 2-hydroxyethyl-trimethylammonium salt of 1-ethyl-3-carboxy-4-oxo-6,7-methylenedioxy-1,4-dihydroquinoline, to a process for the preparation thereof and to pharmaceutical compositions containing it.

The 2-hydroxyethyl-trimethylammonium salt of 1-ethyl-3-carboxy-4-oxo-6,7-methylenedioxy-1,4-dihydroquinoline of the present invention may be prepared by reacting 1-ethyl-3-carboxy-4-oxo-6,7-methylenedioxy-1,4-dihidro-quinoline with 2-hydroxyethyl-trimethylammonium hydroxide. Hereinafter, the salt obtained will be named as choline oxolinate.

It is known that oxolinic acid (chemically 1-ethyl-3-carboxy-4-oxo-6,7-methylenedioxy-1,4-dihydroquinoline) possesses an ureterodisinfecting activity (see the United Kingdom specification No. 1,076,828), however the resorption in the organism of this acid is very disadvantageous because of its low water solubility .

Now it has been found that the solubility of choline oxolinate in water (higher than 1 g/ml) is significantly better than that of oxolinic acid (6,5 $\mu$g/ml). According to our results oxolinic acid does not form any stable salt with other amines, such as triethylamine, dicyclohexylamine or triethanolamine.

On the basis of results of the investigations concerning the solubility, the resorption of choline oxolinate has been supposed to be substantially more advantageous than that of the free oxolinic acid. This supposition was completely verified by the in vivo resorption examinations carried out in mice (see, Example 3.).

The resorption of sodium oxolinate proved to be weaker than that of choline oxolinate of the present invention. · The rapid resorption together with the good bioavailability makes possible on one hand to decrease the effective dose to a large extent, which leads to the diminution of side effects; and to develop a pharmaceutical composition with a systemic action on the other hand. Up to now all the efforts failed to develop a sustained release oxolinic acid form on its slow resorption and rapid eliminations, however this could have been achieved by the choline oxolinate of the present invention because of its more rapid resorption.

Choline oxolinate is siutable for the use in human medicine in the form of tablets, capsules, dragées, coated--dragées, suppositories, solutions, syrups and injections, or in the sustained release form of any of the compositions mentioned above. Choline oxolinate active ingredient in compositions for the veterinary medicine may be used in addition to the forms employed in the human medicine, as active substance alone, or mixed or supported by any form to the feed.

These pharmaceutical compositions can be prepared by the means of methods known in the art.

The present invention is further illustrated in detail by the following Examples without any limitation thereto.

Example 1

Preparation of the 2-hydroxyethyl-trimethylammonium salt of 1-ethyl-3-carboxy-4-oxo-6,7-methylene-dioxy-1,4,-dihydroquinoline (choline oxolinate)

2,61 g (0.01 mole) of 1-ethyl-3-carboxy-4-oxo-6,7-methylenedioxy-1,4-dihydroquinoline were added to a solution containing 2.42 g (0.02 mole) of 2-hydroxyethyl-trimethylammonium hydroxide in 500 ml of water while stirring until the complete dissolution. The solvent was evaporated under reduced pressure, the crystalline residue was disolved in 50 ml of acetone and 4-5 ml of 96 % ethanol were added during constant boiling. After cooling down to 0-5°C, the crystalline precipitate was filtered, washed with 2 x 5 ml of acetone and dried at 80°C under reduced pressure for 2 hours to give 2.63 g (72.3 % yield) of the title product, m.p.: 198 - 200°C (determined by a Boetius melting point apparatus with a heating rate of 4°C/min).

The white, crystalline substance obtained dissolves well in water, ethanol or chloroform, while it is insoluble in acetone or ether.

Characterization of choline oxolinate

a/    Melting point

Choline oxolinate melts at 198 - 200°C, while the melting point of oxolinic acid is 315°C.

b/    Infrared spectrophotometry

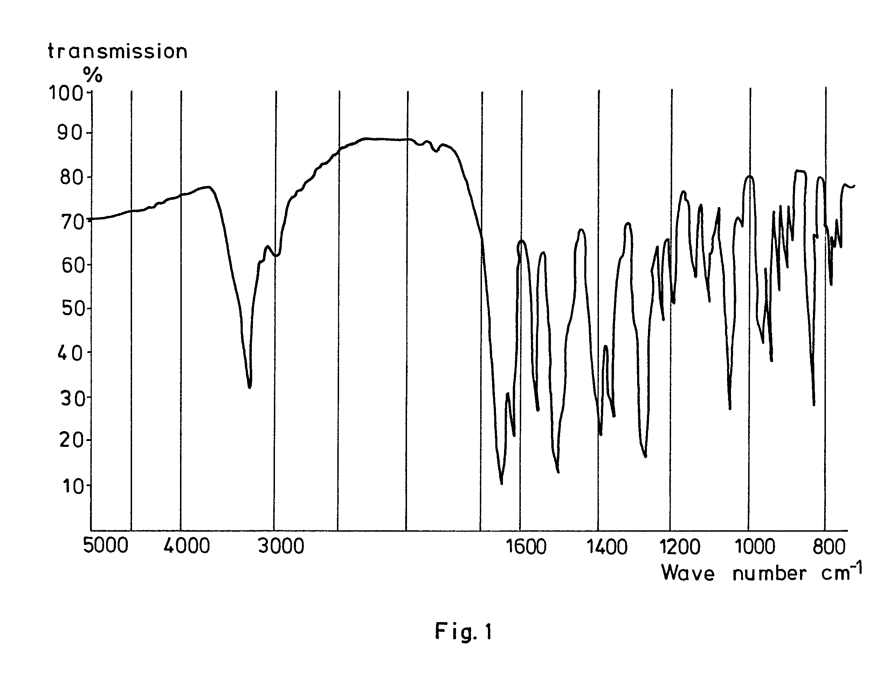

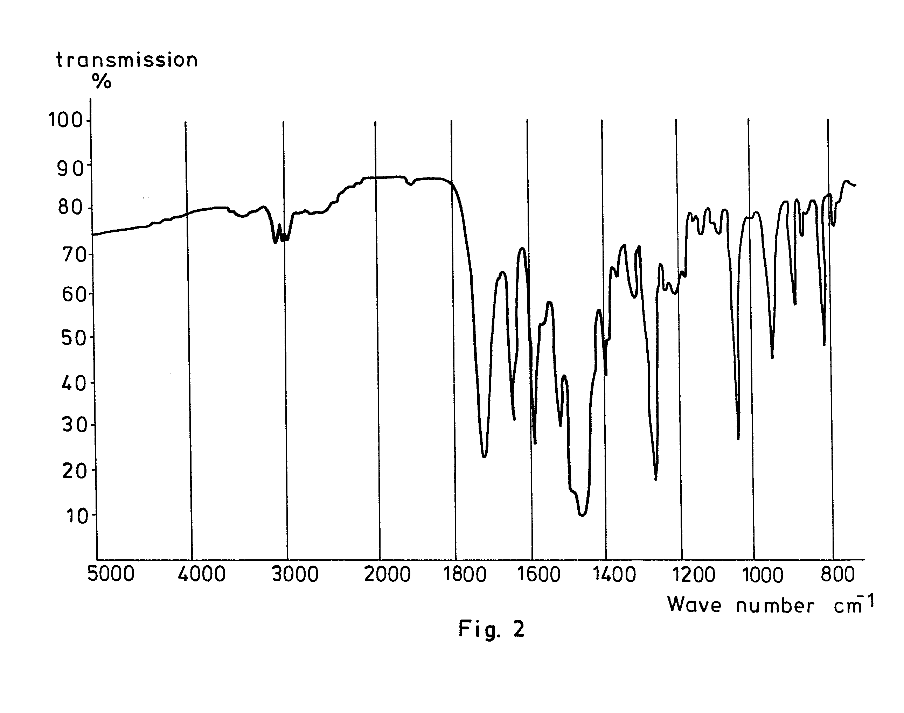

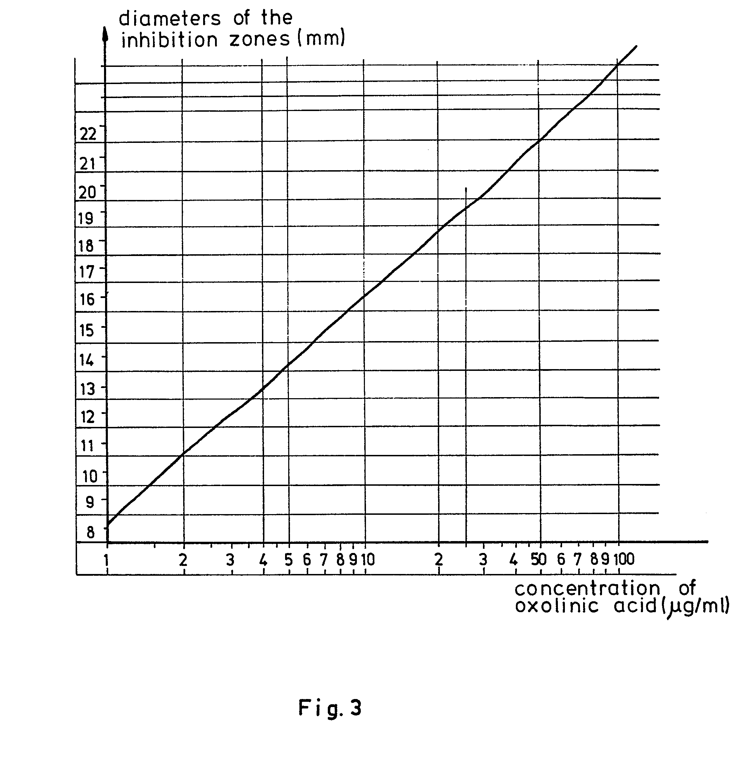

The infrared spectrum of both choline oxolinate (Figure 1) as well as of the starting oxolinic acid (Figure 2) were taken in potassium bromide pellet

- 5 -    0153171

on a Spectromom 2000 type device. It is obvious from comparison of the spectra that two different substances have been investigated.

Example 2

Preparation of choline oxolinate

105 ml of a 50 % solution of 2-hydroxyethyl-trimethylammonium hydroxide (choline base) in methanol were dropped to a suspension containing 100 g of oxolinic acid in 200 ml of methanol stirred in a 1000 ml round-bottom ground-glass flask. The white suspension gradually became brown during the addition of choline base and finally, a dark red, clear solution was obtained. The solution was concentrated by a rotatory evaporating device to give 168 g of a crude product as a viscous oil which was crystallized under cooling. The resulting crude crystalline mass was recrystallized from 120 ml of isopropanol, the separated substance was filtered, washed with 2 x 10 ml of cold isopropanol and the white, crystalline substance was dried at 80°C for 6 hours to give 87.4 g (63 % yield) of the title product.

Example 3

Activity test - Experiments

Male CFLP mice with 25 g of an average body weight were used for these experiments. After starving for 12 hours, the animals were given oxolinic acid or choline oxolinate, respectively in a volume of 2 ml/100 g of body weight as an aqueous suspension containing 1 % of methylcellulose. The doses administered were 50 mg/kg of body weight of oxolinic acid or 70 mg/kg of body weight of choline oxolinate, respectively. )These doses are equimolar as calculated for oxolinic acid.) At 0.5, 1, 2, 3 or 4 hours, respectively after treatment the animals were killed, their blood were centrifuged and the serum were used for the microbiological determination of the concentration.

The examinations were carried out in three parallels. 50 /ul of the serum were applied to an agar gel plate containing Proteus Mirabilis in a concentration of $10^7$ germs/ml. Simultaneously, a calibration curve was taken up with 50 /ul each of suspensions containing oxolinic acid in a concentration of 1, 2, 4, 5, 10, 50 and 100 /ug/ml, respectively. The serum concentrations were calculated from the average of diameters of the inhibition zones by using the calibration curve.

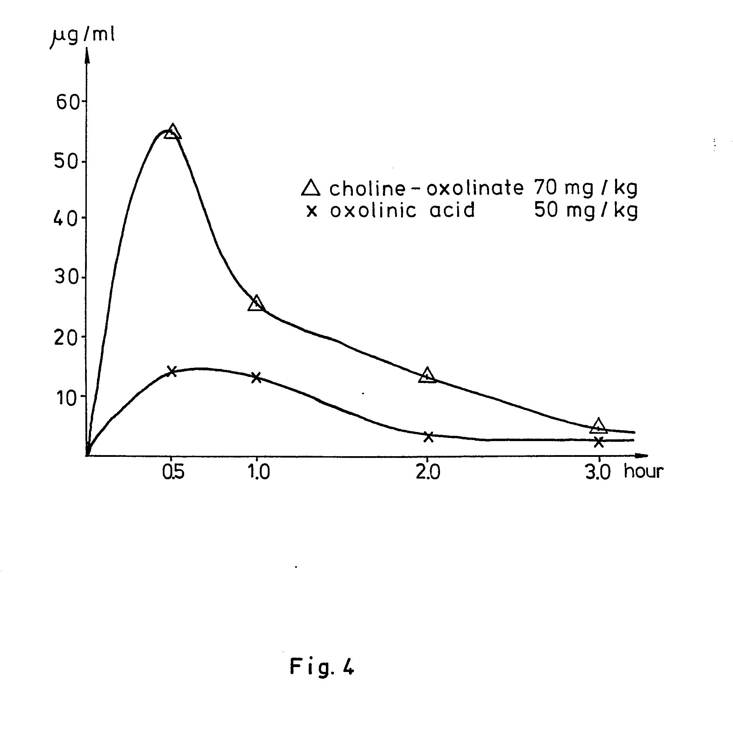

The calibration values are shown in Table I and in Figue 3, respectively. The results of determinations are summarized in Tables II and III and in Figure 4, respectively. Figure 4 shows the development of serum concentration as plotted against the time.

It is obvious from Figure 4 that oxolinic acid is substantially better resorbed from choline oxolinate than after the administration of oxolinic acid. A high level of serum concentration was reached already in 0.5 hour (first sampling) and as an additional advantage, bioavailability is also significantly higher.

## Example 4

### Examples for the formulations

a/ Composition of tablets containing 100 - 300 mg of the active ingredient (as calculated for 100 tablets)

| | |
|---|---|
| Choline oxolinate | 10.0 - 30.0 g |
| Lactose | 2.0 g |
| Saccharose | 0.3 g |
| Potato starch | 5.2 g |
| Mucilago for granulation | q.s. |
| Magnesium stearate | 0.1 g |
| Talc | 0.2 g |
| Potato starch | q.s. to 50.0 g |

### Composition of the granulating liquid

| | |
|---|---|
| White gelatine | 5.0 % |
| Potato starch | 5.0 % |
| Distilled water | q.s. to 100.0 % |

b/ Enteric-coated dragée

The dragée core consisting of the above components was covered with Eudragit L coating by using an appropriate process of dragée preparation.

c/ Composition of sustained release tablets containing 300 mg of the active ingredient (as calculated for 100 tablets).

| | |
|---|---|
| Choline oxolinate | 30.00 g |
| Carboxymethylcellulose sodium salt | 4.50 g |
| Lactose | 5.00 g |
| Gypsum | 48.50 g |
| Stearic acid | 5.00 g |

- 8 -                                    0153171

Distilled water                        q.s.

Magnesium stearate                     0.70 g

Talc                                   1.40 g

  d/ Composition of suppository for children containing 25-50 mg of the active ingredient (as calculated for 100 supporitories).

Choline oxolinate                      2.5 - 5.0 g

Tagat R 1                              7.5        g

Softisan 378                           7.5        g

Witepsol H 32        q.s. to    150.0        g

  e/ Composition of suppository for adults containing 100 - 300 mg of the active ingredient (as calculated for 100 supporitories).

Choline oxolinate                      10.0 - 30.0 g

Tagat R 1                              15.0        g

Softisan 378                           15.0        g

Witepsol H 32        q.s.  to    300.0        g

## Table I

Calibrating series for establishing the correlation

between the serum concentration and inhibition zone

(2 parallels)

| Concentration ($\mu$g/ml) | 100 | 50 | 10 | 5 | 4 | 2 | 1 |
|---|---|---|---|---|---|---|---|
| Diameter of the inhibition zone | 26.5 | 20.7 | 17.9 | 15.0 | 14.4 | 11.6 | 8.6 |
| (mm) | 24.7 | 19.4 | 17.2 | 14.7 | 14.1 | 11.9 | 8.5 |

## Table II

### Diameters of the inhibition zones (mm)

| Sampling (hour) | Animals treated with oxolinic acid | | | Animals treated with choline oxolinate | | |
|---|---|---|---|---|---|---|
| | a | b | c | a | b | c |
| 0.5 | 17.7 | 0 | 18.2 | 22.6 | 22.6 | 21.9 |
| 1.0 | 17.7 | 18.2 | 17.5 | 19.3 | 19.1 | 20.9 |
| 2.0 | 15.3 | 17.4 | 8.8 | 18.2 | 17.8 | 17.8 |
| 3.0 | 14.0 | 16.0 | 14.3 | 13.9 | 15.3 | 11.4 |
| 4.0 | 16.8 | 14.7 | 14.1 | 11.7 | 11.7 | 14.3 |

## Table III

### Plasma concentrations from the calibration curve
### ($\mu$g/ml)

| Time (hour) | 0.5 | 1 | 2 | 3 | 4 |
|---|---|---|---|---|---|
| Animals treated with oxolinic acid | 15.0 | 14.5 | 4.6 | 4.2 | 4.8 |
| Animals treated with choline oxolinate | 56.0 | 26.0 | 15.0 | 6.6 | 3.1 |

CLAIMS:

1/ 2-Hydroxyethyl-trimethylammonium salt of 1-ethyl-3-carboxy-4-oxo-6,7-methylenedioxy-1,4-dihydroquinoline.

2/ A process for the preparation of 2-hydroxyethyltrimethylammonium salt of 1-ethyl-3-carboxy-4-oxo-6,7-methylenedioxy-1,4-dihydroquinoline, which comprises reacting 1-ethyl-3-carboxy-4-oxo-6,7-methylenedioxy-1,4-dihydroquinoline with an aqueous or ethanolic or aqueous-ethanolic solution of 2-hydroxyethyl-trimethylammonium hydroxide.

3/ A pharmaceutical composition, which comprises the 2-hydroxyethyl-trimethylammonium salt of 1-ethyl-3-carboxy-4-oxo-6,7-methylenedioxy-1,4-dihydroquinoline as active ingredient and optionally an inert, non-toxic diluent or carrier material conventionally used in the human and veterinary medicine.

4/ A pharmaceutical composition according to claim 3, which comprises the active ingredient in the form of tablet, capsule, dragée, coated-dragée, suppository, solution, syrup or injection, or in the sustained release form of any of said forms for the human medicine, and, in addition to said forms, the active ingredient alone or mixed or supported in any form with the feed of animals for veterinary medicine.

Fig. 1

Fig. 2

diameters of the
inhibition zones (mm)

concentration of
oxolinic acid (µg/ml)

Fig. 3

Fig. 4